# EUROPEAN PATENT APPLICATION

(11) **EP 2 676 618 A1**
(43) Date of publication of application: **25.12.2013**
(21) Application number: 11858968.8
(22) Date of filing: 10.11.2011
(51) Int. Cl.: A61B 17/34

(54) **VERESS NEEDLE**

(30) Priority: 18.02.2011 JP 2011033414
(71) Applicant: Terumo Kabushiki Kaisha, Tokyo 151-0072 (JP)
(72) Inventor: KAWAURA, Masakatsu, Ashigarakami-gun, Kanagawa 259-0151 (JP)
(74) Representative: Dossmann, Gérard
(86) International application number: PCT/JP2011/075934
(87) International publication number: WO 2012/111201

(57) **Abstract**

[PURPOSE]

To provide a Veress needle which can easily prevent a needle tip from being pulled out from a puncturing site.

[CONSTITUTION]

A Veress needle including a hollow puncturing needle 110 having a distal end portion with a needle tip 112 and a lumen 118, an insertion member 130 slidably inserted in the lumen 118, an urging device and a pullout preventing device 105. The urging device is adapted to urge the insertion member 130 such that, before puncturing is performed by the needle tip 112 and after the puncturing is completed, a distal end portion 134 of the insertion member 130 protrudes from the needle tip 112, and the distal end portion 134 of the insertion member 130 is retreated to the lumen 118 based on a load of the puncturing upon the puncturing by the needle tip 112. The pullout preventing device 105, after the load of the puncturing disappears, prevents the needle tip 112 from being pulled out from a puncturing site in conjunction with protruding movement of the distal end portion 134 of the insertion member 130 from the lumen 118.

## Description

### Technical Field of the Invention

The present invention relates to a Veress needle.

### Background of the Invention

When, for example, it is necessary to puncture an abdominal wall or a vaginal wall, a Veress needle with a guarded needle tip is applied to prevent damages on organs positioned on an inner side of a puncturing site (see, for example, Patent Reference 1 and Patent Reference 2).

### Prior Art Documents

### Patent reference

[Patent reference 1] JP 5-130999 A
[Patent reference 2] JP 9-135841 A

### Summary of Invention

### Problems to be Solved by the Invention

However, there is aproblemthat it is difficult to prevent a needle tip from being pulled out from a puncturing site. When, for example, the needle tip is prevented from being pulled out by way of deep puncturing, there is a possibility that organs are damaged even if the needle tip is guarded, and an operator pays significant attention to puncturing to prevent such a situation and therefore operability is very poor.

The present invention is made in order to solve the abovementioned problem associated with the related art, and to provide a Veress needle which can easily prevent a needle tip from being pulled out from a puncturing site.

### Means to Solve the Problems

This invention for accomplishing the above-mentioned purpose is a Veress needle comprising a hollow puncturing needle which comprises a distal end portion with a needle tip and a lumen, an insertion member which is slidably inserted in the lumen, an urging device, and a pullout preventing device. The urging device is adapted to urge the insertion member such that, before puncturing is performed by means of the needle tip and after the puncturing is completed, a distal end portion of the insertion member protrudes from the needle tip and, the distal end portion of the insertion member is retreated to the lumen based on a load of the puncturing upon the puncturing by means of the needle tip. The pullout preventing device, after the load of the puncturing disappears, prevents the needle tip from being pulled out from a puncturing site in conjunction with protruding movement of the distal end portion of the insertion member from the lumen.

### Effects of the Invention

Since a pullout preventing device which prevents a needle tip (puncturing needle) from being pulled out from a puncturing site after the load of the puncturing disappears and puncturing is completed is provided, it is not necessary to prevent the needle tip from being pulled out by way of deep puncturing, for example. Further, the needle tip is prevented from being pulled out in conjunction with protruding movement of the distal end portion of the insertion member from the lumen of the puncturing needle, so that operability is good. Consequently, it is possible to provide a Veress needle which can easily prevent the needle tip from being pulled out from the puncturing site.

Further, when a locking device which is adapted to lock the movement of the insertion member in an urging direction to define an initial position of the distal end portion of the insertion member before the puncturing by means of the needle tip, and, when the distal end portion of the insertion member retreats to the lumen, unlocks the lock and releases an urging force of the urging device is provided in an operating unit which is positioned on a proximal end portion of the puncturing needle and at which the urging device is disposed, the pullout preventing device can prevent the needle tip from being pulled out from the puncturing site based on the protruding movement of the distal end portion of the insertion member beyond the initial position due to the released urging force after the load of the puncturing disappears. In other words, a mechanism which interlocks the protruding movement of the distal end portion of the insertion member from the lumen of the puncturing needle and the prevention motion of the needle tip from being pulled out from the puncturing site can be accomplished by the locking device which adopts a simple structure.

The locking device may also comprises a hook which locks the movement of the insertion member in the urging direction by means of engaging with the insertion member, and unlocks the lock by means of moving away from the insertion member based on retreating movement of the distal end portion of the insertion member to the lumen. In this case, a mechanism which can lock movement of the insertion member can be accomplished by a simple structure.

In addition to the hook, the locking device can further comprise: a sliding portion which is disposed closer to the proximal end portion side than an extended portion which is disposed closer to a proximal end portion side than the hook in a proximal end portion of the insertion member and which protrudes in a direction which crosses an axial direction of the insertion member, and which is driven by abutting a proximal end portion side surface of the extended portion which moves based on the retreating movement of the distal end portion of the insertion member to the lumen, and moves along the proximal end portion of the insertion member; and an arm which is extended from an outer periphery of the operating unit at the proximal end portion, and which connects the sliding portion and the hook through an opening formed in the operating unit at the proximal end portion. In this case, the hook engages with the insertion member by means of abutting on a distal end portion side surface of the extended portion, and moves accompanying the movement of the sliding portion, and then is separated from the insertion member and disengages the engagement. Consequently, a mechanism which unlocks the lock based on the retreating movement of the distal end portion of the insertion member to the lumen can be accomplished by a simple structure.

When the distal end portion of the insertion member which is adapted to be in a bent shape, and be inserted in the lumen in an elastically deformed state, protrude from the lumen after the puncturing is completed, then be restored to the bent shape before elastic deformation and be caught on an inner surface of the puncturing site forms the pullout preventing device, the pullout preventing device can adopt a simple structure.

When the insertion member is hollow and comprises a lumen in which a second insertion member is inserted, the distal end portion of the insertionmember comprises a lateral opening which communicates with the lumen, and a distal end portion of the second insertion member which is adapted to be in a bent shape, and be inserted in the lumen of the insertion member in an elastically deformed state, be extended from the lateral opening of the insertion member which protrudes from the lumen of the puncturing needle after the load of the puncturing disappears, then be restored to the bent shape before elastic deformation and be caught on the inner surface of the puncturing site forms the pullout preventing device, the pullout preventing device can also adopt a simple structure.

When the insertion member is detachably attached to the operating unit at the proximal end portion, it is possible to improve the degree of freedom upon use.

When a hollow tubular sleeve in which the puncturing needle is slidably inserted is detachably attached to the operating unit at the proximal end portion, it is possible to indwell the tubular sleeve in the puncturing site by detaching the tubular sleeve from the operating unit at the proximal end portion (releasing the connection to the operating unit at the proximal end portion) after puncturing is completed. In other words, the tubular sleeve can form an outer needle of an indwelling needle and the puncturing needle can form an inner needle of the indwelling needle.

At the operating unit at the proximal end portion, it is also possible to dispose a checking device for checking completion of the puncturing visually. In this case, it is possible to improve operability.

The checking device may be formed by an end surface of the proximal end portion of the insertion member, the end surface adapted to protrude outward from a proximal end portion of the operating unit at the proximal end portion when being at the initial position, and be accommodated in the operating unit at the proximal end portion when the puncturing is completed. In this case, the checking device can adopt a simple structure.

The insertion member may be formed by a guide wire which is adapted to be separably from the puncturing needle and the operating unit at the proximal end portion. In this case, it is possible to securely indwell the guide wire in the puncturing site.

The extended portion at the proximal end portion of the insertionmembermaybe formed by a detachable different member. In this case, interference of the extendedportion is prevented by detaching the extended portion from the proximal end portion of the insertion member, so that it is possible to easily separate the insertion member from the puncturing needle and the operating unit at the proximal end portion.

The objects, features, and characteristics of this invention other than those set forth above will become apparent from the description given herein below with reference to preferred embodiments illustrated in the accompanying drawings.

### Brief Explanation of the Drawings

Fig. 1 is a side view of assistance in explaining a Veress needle according to a first embodiment.
Fig. 2 is a side view of assistance in explaining a tubular sleeve illustrated in Fig. 1.
Fig. 3 is a side view of assistance in explaining a puncturing needle illustrated in Fig. 1.
Fig. 4 is a plan view of assistance in explaining a distal end portion illustrated in Fig. 1.
Fig. 5 is a cross-sectional view of assistance in explaining the distal end portion illustrated in Fig. 1.
Fig. 6 is a plan view of assistance in explaining an operating unit at a proximal end portion illustrated in Fig. 1.
Fig. 7 is a cross-sectional view of assistance in explaining the operating unit at the proximal end portion illustrated in Fig. 1.
Fig. 8 is a perspective view of assistance in explaining a locking device illustrated in Fig. 6.
Fig. 9 is a cross-sectional view of assistance in explaining a method of using the Veress needle according to the first embodiment, and illustrates the distal end portion upon start of puncturing.
Fig. 10 is a cross-sectional view of assistance in explaining the operating unit at the proximal end portion upon start of puncturing.
Fig. 11 is a cross-sectional view of assistance in explaining the distal end portion upon completion of puncturing.
Fig. 12 is a cross-sectional view of assistance in explaining the operating unit at the proximal end portion upon completion of puncturing.
Fig. 13 is a cross-sectional view of assistance in explaining how the tubular sleeve is indwelled.
Fig. 14 is a cross-sectional view of assistance in explaining Modified Example according to the first embodiment.
Fig. 15 is a cross-sectional view of assistance in explaining a distal end portion of a Veress needle according to a second embodiment.
Fig. 16 is a cross-sectional view of assistance in explaining an operating unit at a proximal end portion of the Veress needle according to the second embodiment.
Fig. 17 is a cross-sectional view of assistance in explaining a distal end portion of a Veress needle according to a third embodiment.
Fig. 18 is a cross-sectional view of assistance in explaining an operating unit at a proximal end portion of the Veress needle according to the third embodiment.
Fig. 19 is a cross-sectional view of assistance in explaining a method of using the Veress needle according to the third embodiment, and illustrates the distal end portion upon start of puncturing.
Fig. 20 is a cross-sectional view of assistance in explaining the operating unit at the proximal end portion upon start of puncturing.
Fig. 21 is a cross-sectional view of assistance in explaining the distal end portion upon completion of puncturing.
Fig. 22 is a cross-sectional view of assistance in explaining the operating unit at the proximal end portion upon completion of puncturing.
Fig. 23 is a cross-sectional view of assistance in explaining separation of the operating unit at the proximal end portion.
Fig. 24 is a cross-sectional view of assistance in explaining separation of a fixing connector.
Fig. 25 is a cross-sectional view of assistance in explaining how a guide wire is indwelled.

### Working Example of Carrying Out the Invention

Hereinafter, embodiments of the present invention will be described referring to the drawings.

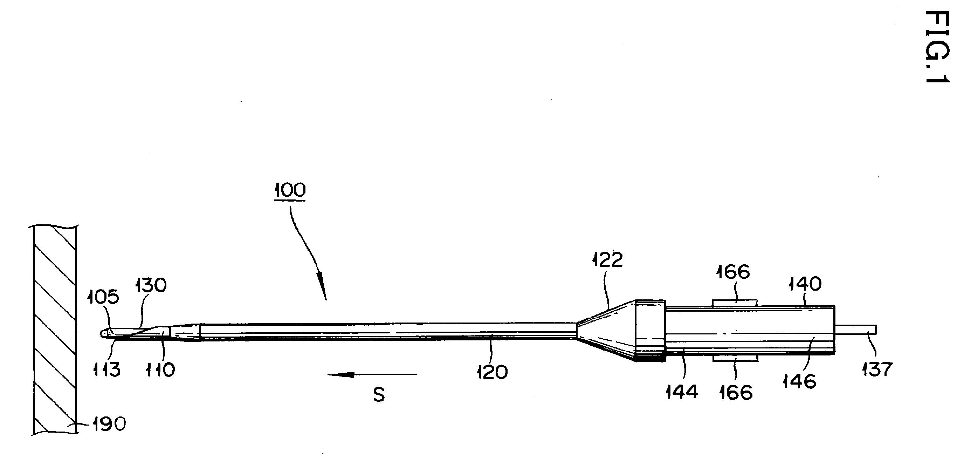

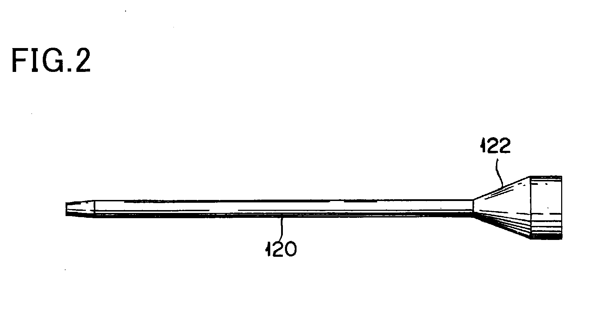

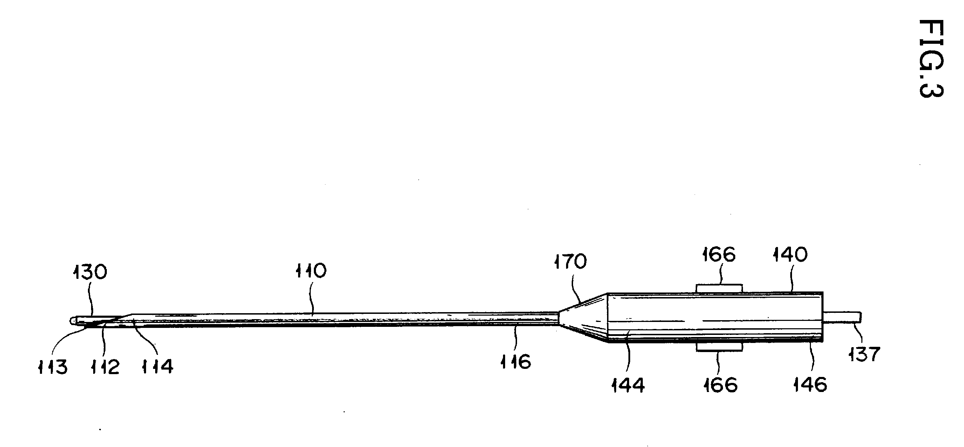

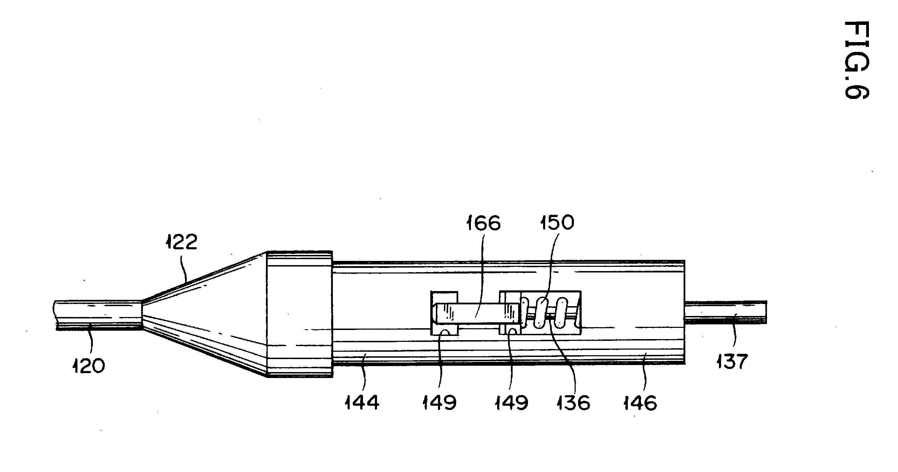

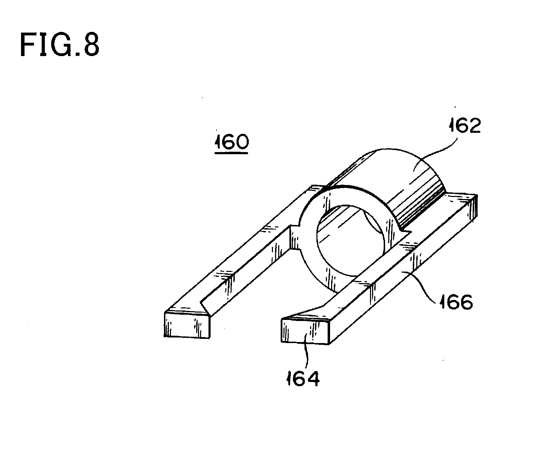

Fig. 1 is a side view of assistance in explaining a Veress needle according to a first embodiment, Fig. 2 is a side view of assistance in explaining a tubular sleeve illustrated in Fig. 1, Fig. 3 is a side view of assistance in explaining a puncturing needle illustrated in Fig. 1, Figs. 4 and 5 are a plan view and a cross-sectional view of assistance in explaining a distal end portion illustrated in Fig. 1, Figs. 6 and 7 are a plan view and a cross-sectional view of assistance in explaining an operating unit at a proximal end portion illustrated in Fig. 1 and Fig. 8 is a perspective view of assistance in explaining a locking device illustrated in Fig. 6.

As illustrated in Fig. 1, a Veress needle 100 according to the first embodiment has a puncturing needle 110, a tubular sleeve 120, an insertion member 130, a pullout preventing device 105 and an operating unit 140 at the proximal end portion, and is used to puncture a wall portion 190 of a living body. The wall portion 190 is a vaginal wall when the Veress needle is applied to the transvaginal hydrolaparoscopy (THL). The wall portion 190 is not limited to the vaginal wall, and may also be an abdominal wall.

The puncturing needle 110 is a hollow tube, and has a distal end portion 114 at which a sharp needle tip 112 is formed and a proximal end portion 116 to which a needle hub 170 is connected (see Fig. 3). The needle hub 170 has a hollow shape, and is used to connect the proximal end portion 116 of the puncturing needle 110 to the operating unit 140 at the proximal end portion.

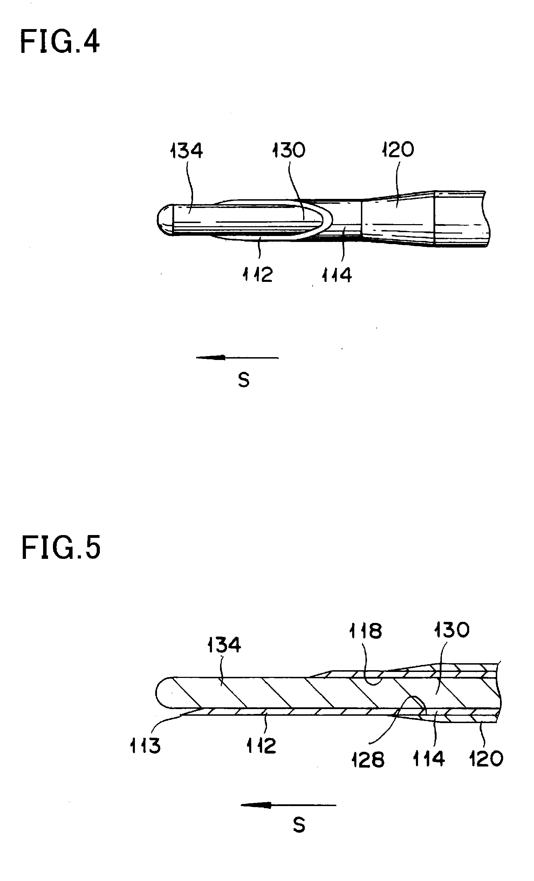

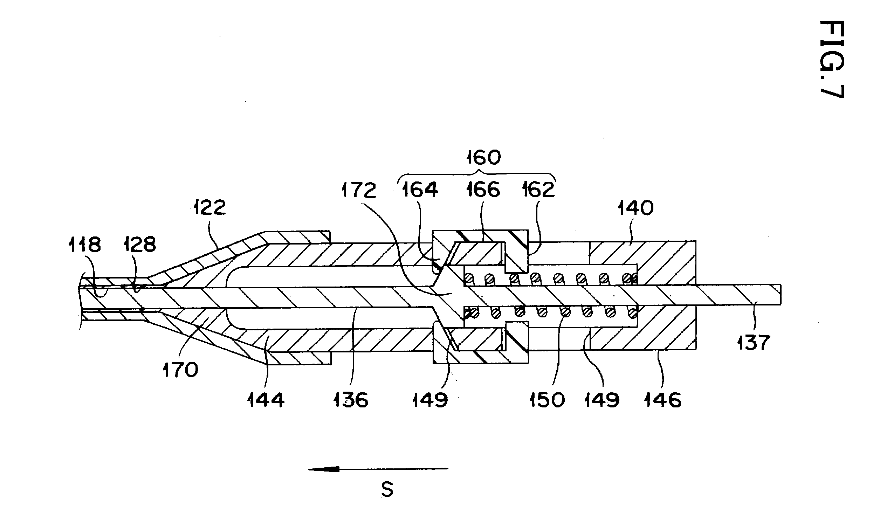

The tubular sleeve 120 (see Fig. 2) has a lumen 128 in which the puncturing needle 110 is inserted (see Figs. 5 and 7), and a proximal end portion at which a sleeve hub 122 for connecting the operating unit 140 at the proximal end portion is disposed. The outer hub 122 is positioned on an outer side of the needle hub 170, and is detachably attached (connected) to the operating unit 140 at the proximal end portion. The tubular sleeve 120 is detached from the operating unit 140 at the proximal end portion when puncturing is completed, and is indwelled in a puncturing site. In other words, the tubular sleeve 120 forms an outer needle of an indwelling needle and the puncturing needle 110 forms an inner needle of the indwelling needle.

The insertion member 130 has a distal end portion 134 which is slidably (movably in an axial direction S) inserted in the lumen 118 of the puncturing needle 110 and which protrudes from the distal end portion 114 of the puncturing needle 110, and a proximal end portion 136 which extends inside the operating unit 140 at the proximal end portion. The distal end portion 134 is formed to guard the needle tip 112 to prevent a damage on an organ positioned on an inner side of a puncturing site of the wall portion 190 (see Figs. 4 and 5). The proximal end portion 136 has an extended portion 172 which protrudes in a direction which crosses the axial direction S of the insertion member 130, and the extended portion 172 is integrally formed by the proximal end portion 136 (see Fig. 7).

The pullout preventing device 105 is used to prevent the needle tip 112 from being pulled out from the puncturing site in conjunction with protruding movement of the distal end portion 134 of the insertion member 130 from the lumen 118 of the puncturing needle 110 after a load of the puncturing disappears.

The operating unit 140 at the proximal end portion has a distal end portion 144 which is positioned on a proximal end portion side of the puncturing needle 110 and which is integrally formed with the needle hub 170, a cap portion 146 which is positioned at the proximal end portion and an outer periphery portion at which an opening 149 is disposed, and includes an urging device 150 and a locking device 160 disposed therein (see Figs. 6 and 7).

The proximal end portion 136 of the insertion member 130 penetrates the cap portion 146, and an end surface 137 of the proximal end portion 136 which protrudes outward is adapted to be accommodated inside the operating unit 140 at the proximal end portion when puncturing is completed, so that it is possible to check that puncturing is completed, based on a change in the position of the end surface 137. In other words, a length of the proximal end portion 136 of the insertion member 130 is set to enable completion of puncturing to be visually checked, and the end surface 137 which protrudes outward from the cap portion 146 forms a checking device for visually checking completion of the puncturing. Consequently, it is possible to improve operability, and make a structure of the checking device simple.

The urging device 150 is formed by an elastic body (spring) which urges the extended portion 172 of the proximal endportion 136 of the insertion member 130 towards the distal end portion side, and is disposed between the extended portion 172 and the cap portion 146. The urging device 150 is adapted to urge the insertion member 130 such that, before puncturing is performed by means of the needle tip 112 (in a state in which the load of puncturing does not apply) and after the load of the puncturing disappears, the distal end portion 134 of the insertion member 130 protrudes from the needle tip 112, and the distal end portion 134 of the insertion member 130 is retreated to the lumen 118 of the puncturing needle 110 by the load of puncturing upon puncturing by means of the needle tip 112.

The locking device 160 is adapted to lock movement of the insertion member 130 in the urging direction in the lumen 118 of the puncturing needle 110 to define an initial position of the distal end portion 134 of the insertion member 130 before puncturing is performed by means of the needle tip 112, and unlock the lock when the distal end portion 134 of the insertion member 130 is retreated to the lumen 118 of the puncturing needle 110. The urging direction matches with the axial direction S leading to the distal end portion. In addition, the distal end portion 134 of the insertion member 130 is set at an initial position to slightly protrude from the leading end of a bevel (blade surface) of the needle tip 112 of the puncturing needle 110, and guard the needle tip 112 (see Figs. 4 and 5).

Specifically speaking, the locking device 160 has a sliding portion 162, hooks 164 and arms 166 as illustrated in Fig. 8.

The sliding portion 162 has a cylindrical shape, is disposed closer to the proximal end portion side than the extended portion 172 of the proximal end portion 136 of the insertion member 130, is driven by abutting a proximal end portion side surface of the extended portion 172 on the sliding portion 162 and moves along the proximal end portion 136 of the insertion member 130. The proximal end portion side surface of the extended portion 172 moves based on retreating movement of the distal end portion 134 of the insertion member 130 to the lumen 118 of the puncturing needle 110.

The hooks 164 lock movement of the insertion member 130 in the urging direction S when engaging with the insertion member 130, and unlock the lock when separated from the insertion member 130 based on the retreating movement of the distal end portion 134 of the insertion member 130 to the lumen 118 of the puncturing needle 110. In the present embodiment, the hooks 164 have claw shapes, are disposed closer to the distal end portion side than the extended portion 172, abut on and engage with the distal end portion side surface of the extended portion 172 and lock movement of the insertion member 130 in the urging direction (in the axial direction S leading to the distal end portion).

The arms 166 are extended from the outer periphery of the distal end (needle hub 170) of the operating unit 140 at the proximal end portion, and connects the sliding portion 162 and the hooks 164 through the opening 149 of the operating unit 140 at the proximal end portion.

The hooks 164 are adapted to, by moving accompanying the movement of the sliding portion 162 upon movement of the sliding portion 162, be pulled out from the opening 149 (separated from the insertion member 130), disengage the engagement, be elastically deformed and abut (get on) the outer periphery of the operating unit 140 at the proximal end portion.

Since the hooks 164 do not abut on the distal end portion side surface of the extended portion 172 of the proximal end portion 136 of the insertion member 130 when the distal end portion 134 of the insertion member 130 protrudes from the lumen 118 of the puncturing needle 110 after the load of the puncturing disappears, a force of the urging device 150 contracted on the proximal end side is released and the distal end portion 134 of the insertion member 130 protrudes beyond the initial position.

In addition, protrusion of the insertion member 130 is arrested (stopped) by abutting the extended portion 172 on an inner surface of the distal end portion 144 of the operating unit 140 at the proximal end portion. Hence, the amount of protrusion of the insertion member 130 is defined by a distance between the distal end side surface of the extended portion 172 and the inner surface of the distal end portion 144 of the operating unit 140 at the proximal end portion at the initial position.

The pullout preventing device 105 according to the present embodiment is formed by the distal end portion 134 of the insertion member 130, and the distal end portion 134 is adapted to be in a bent shape (J-shaped structure under natural circumstances), be inserted in the lumen 118 of the puncturing needle 110 in an elastically deformed state, protrudes from the lumen 118 of the puncturing needle 110 after the load of the puncturing disappears, then be restored to the bent shape before elastic deformation and is caught on an inner surface of the puncturing site. In other words, after the load of the puncturing disappears and puncturing is completed, the needle tip 112 is prevented from being pulled out from the puncturing site based on protruding movement of the unlocked distal end portion 134 of the insertion member 130 beyond the initial position. Consequently, the pullout preventing device 105 can be formed by a simple structure.

In addition, in order to securely guard the needle tip 112, it is preferable that the distal end portion 134 of the insertion member 130 is formed to bend toward a bevel leading end side 113 of the needle tip 112. This can be accomplished by making a setting to impart a directional character to a cross-sectional shape of the distal end portion 134 of the insertion member 130 and a cross-sectional shape of the lumen 118 of the distal end portion 114 of the puncturing needle 110 and prevent the distal end portion 134 of the insertion member 130 from moving from an orientation position at which bending toward the bevel leading end side 113 is made (the distal end portion 134 of the insertion member 130 from rotating). For example, the directional character can be obtained by forming depressions and protrusions in an outer periphery of the insertion member 130 along an extension direction of the insertion member 130 and forming in an inner surface of the lumen 118 of the puncturing needle 110 depressions and protrusions which fit to the above depressions and the protrusions. Further, it is also possible to obtain the directional character by making the cross-sectional shapes in shapes having directional character (for example, rectangular shapes or triangular shapes).

Since the Veress needle 100 has the pullout preventing device which prevents a needle tip (puncturing needle) from being pulled out from a puncturing site after the load of the puncturing disappears and puncturing is completed as described above, it is not necessary to prevent the needle tip from being pulled out by way of, for example, deep puncturing. Further, the needle tip is prevented from being pulled out in conjunction with protruding movement of the distal end portion of the insertion member from the lumen of the puncturing needle, so that operability is good.

Furthermore, a mechanism which interlocks the protruding movement of the distal end portion of the insertion member from the lumen of the puncturing needle and the prevention motion of the needle tip from being pulled out from the puncturing site can be accomplished by a simple structure.

Still further, a mechanism which can lock movement of the insertion member and a mechanism which unlocks the lock based on the retreating movement of the distal end portion of the insertion member to the lumen can be accomplished by simple structures.

As materials for forming the puncturing needle 110, the tubular sleeve 120 and the insertion member 130, flexible metals, polymer materials with relatively high rigidity or suitable combinations of these are cited. For example, the metal includes stainless steel, Ni-Ti alloy, Cu-Zn alloy, cobalt alloy and tantalum, and the polymer materials include polyamide, polyimide, ultrahigh molecular weight polyethylene, polypropylene and fluorine resin.

As materials for forming the sleeve hub 122 and the needle hub 170, thermoplastic resins such as polycarbonate, polyamide, polysulfone, polyarylate, and methacrylate-butylene-styrene copolymer is cited.

Next, a method of using the Veress needle according to the first embodiment will be described.

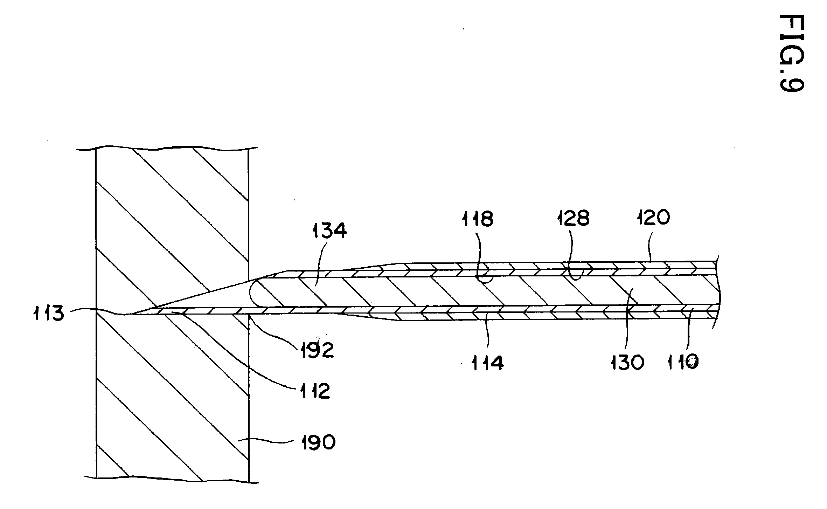

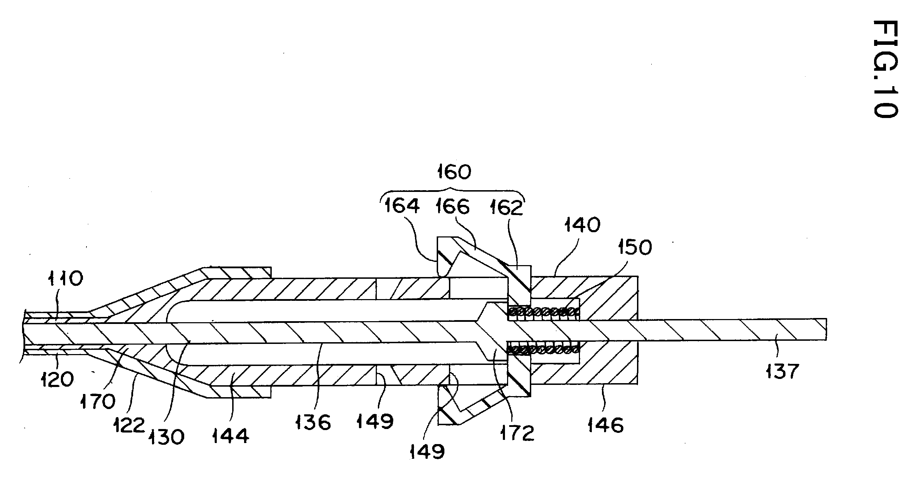

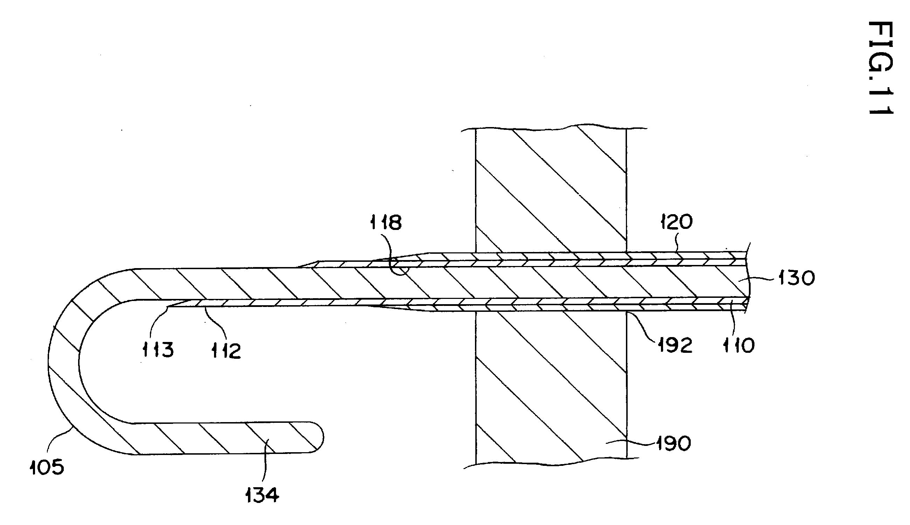

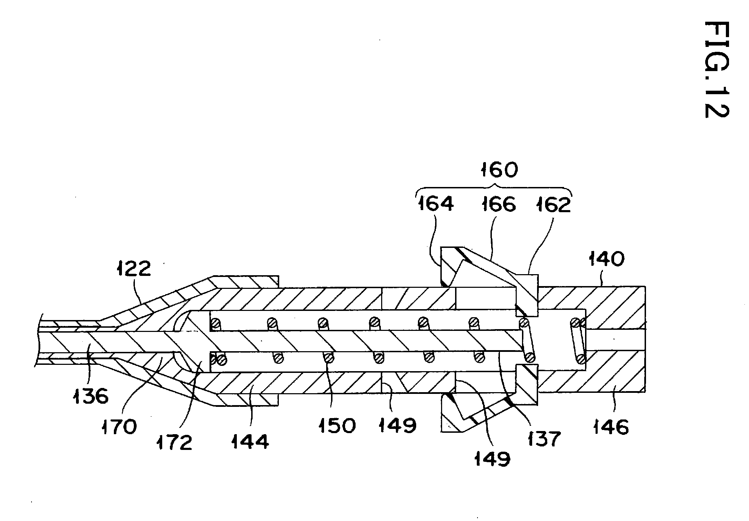

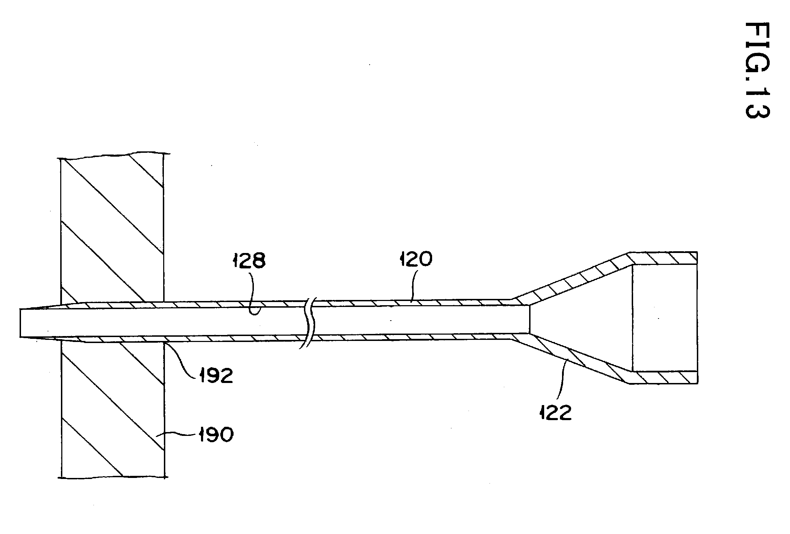

Figs. 9 and 10 are cross-sectional views of assistance in explaining the distal end portion and the operating unit at the proximal end portion upon start of puncturing, Figs. 11 and 12 are cross-sectional views of assistance in explaining the distal end portion and the operating unit at the proximal end portion upon completion of puncturing, and Fig. 13 is a cross-sectional view of assistance in explaining how the tubular sleeve is indwelled.

When the Veress needle is applied to the transvaginal hydrolaparoscopy, disinfection of an external genitalia and the inside of a vagina, insertion of an ultrasonic probe and expansion of a vaginal fornix by means of Cusco speculum, etc. are performed as preliminary arrangements.

Then, a distal end portion of the Veress needle 100 is inserted in the vagina. At this point, the distal end portion 134 of the insertion member 130 protrudes from the distal end portion 114 of the puncturing needle 110 based on an urging force of the urging device 150, and guards the needle tip 112 (see Fig. 5). Meanwhile, the hook 164 of the locking device 160 disposed at the operating unit 140 at the proximal end portion abuts on the distal end portion side surface of the extended portion 172 of the proximal end portion 136 of the insertionmember 130 and, thus, locks movement of the insertion member 130 in the urging direction (in the axial direction S leading to the distal end portion) (see Fig. 7). In other words, the distal end portion 134 of the insertion member 130 is retained in an elastically deformed state (straight state) since being at the initial position although slightly protruding from the bevel leading end side 113 of the needle tip 112 of the puncturing needle 110.

Further, when abutting on the wall portion 190, the distal end portion 134 of the insertion member 130 retreats to the lumen 118 of the puncturing needle 110 against the urging force of the urging device 150 based on a resistance resulting from the aforementioned abutting. Thereby, the distal end portion 114 of the puncturing needle 110 is exposed and, as illustrated in Fig. 9, the needle tip 112 punctures the wall portion 190 of the vaginal wall.

At this point, the proximal end portion side surface of the extended portion 172 of the proximal end portion 136 of the insertion member 130 abuts on the sliding portion 162 of the locking device 160, and moves (drives) the sliding portion 162 along the proximal end portion 136 of the insertion member 130. Thereby, the hooks 164 of the locking device 160 move accompanying the sliding portion 162, are pulled out from the opening 149 (separated from the insertion member 130), are elastically deformed, and abut on (get on) the outer periphery of the operating unit 140 at the proximal end portion. Thus, the lock of the extended portion 172 of the proximal end portion 136 of the insertion member 130 is unlocked as illustrated in Fig. 10.

Then, when the needle tip 112 penetrates the wall portion 190, the resistance resulting from the abutting on the wall portion 190 disappears, and the distal end portion 134 of the insertion member 130 protrudes from the distal end portion 114 of the puncturing needle 110 based on the urging force of the urging device 150 and guards the needle tip 112. Consequently, it is possible to prevent the damage on the organ positioned on the inner side of the puncturing site 192 of the wall portion 190.

At this point, the lock of the extended portion 172 of the proximal end portion 136 of the insertion member 130 by means of the hook 164 of the locking device 160 is unlocked (the hook 164 does not abut on the distal end portion side surface of the extended portion 172). Thus, the force of the urging device 150 contracted on the proximal end side is released and the extended portion 172 moves to abut on the inner surface of the distal end portion 144 of the operating unit 140 at the proximal end portion as illustrated in Fig. 12.

Thereby, the distal end portion 134 of the insertion member 130 protrudes beyond the initial position, and, as illustrated in Fig. 11, is restored to the bent shape before elastic deformation and is caught on the inner surface of the puncturing site 192, so that the needle tip 112 is prevented from being pulled out from the puncturing site 192.

Further, although the end surface 137 of the proximal end portion 136 of the insertion member 130 protrudes outward from the cap portion 146 of the operating unit 140 at the proximal end portion while puncturing is performed halfway (see Fig. 10), the end surface 137 is accommodated inside the operating unit 140 at the proximal end portion when puncturing is completed (see Fig. 12). Consequently, it is possible to visually check that puncturing is completed based on a change in the position of the end surface 137.

Next, the sleeve hub 122 and the operating unit 140 at the proximal end portion are disconnected, and the operating unit 140 at the proximal end portion is retreated in a state in which the position of the tubular sleeve 120 is fixed. Thereby, the distal end portion 134 of the insertion member 130 is elastically deformed by the resistance generated by abutting on the wall portion 190, becomes straight and passes through the puncturing site 192. Meanwhile, as illustrated in Fig. 13, the tubular sleeve 120 is indwelled in the puncturing site 192, and the tubular sleeve 120 forms the outer needle of the indwelling needle. In addition, the puncturing needle 110 forms the inner needle of the indwelling needle.

The tubular sleeve 120 which is indwelled in the puncturing site 192 (functions as the indwelling needle) is used for injection of physiological salt solution, insertion of an endoscope and medical treatment, etc.

For example, the physiological salt solution is injected between the uterus and the rectum through a cannula inserted in the lumen 128 of the tubular sleeve 120 to float the uterine tube and the ovary. For example, the endoscope is inserted to inspect (observe) a surface of the floated uterine tube and ovary after the injection of the physiological salt solution is completed and the cannula is taken out. Inspection items include tubal patency, adhesion around the uterine tube and whether or not an endometriosis develops.

A medical treatment target is, for example, an endometriosis externa (heterotopic endometriosis), and a needle is inserted to puncture and suction a chocolate cyst.

Further, after use, bleeding from the puncturing site 192 is stopped as after-treatment. In addition, a treatment tool inserted in the lumen 128 of the tubular sleeve 120 is not limited to a cannula, an endoscope and a needle for puncturing and suctioning.

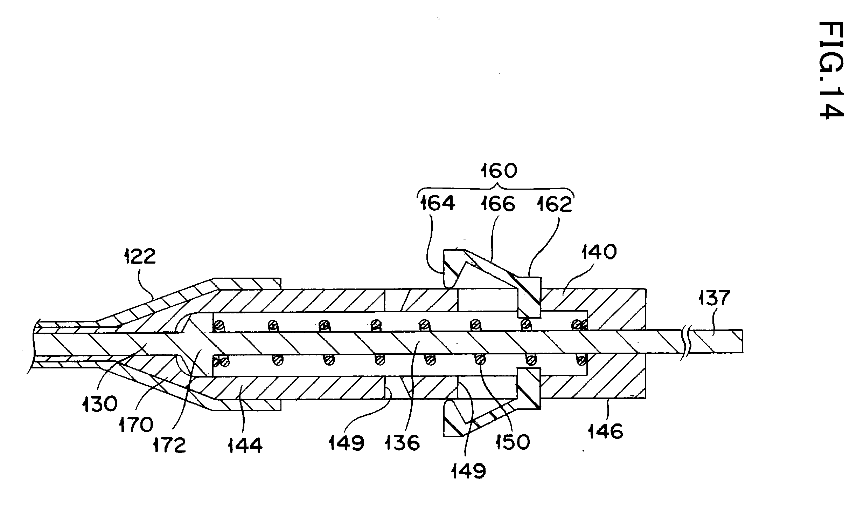

Fig. 14 is a cross-sectional view of assistance in explaining Modified Example according to the first embodiment.

It is also possible to extend the proximal end portion 136 of the insertion member 130 over the length from the extended portion 172 to the end surface 137, and keep the end surface 137 protruding from the cap portion 146 of the operating unit 140 at the proximal end portion even when puncturing is completed. In this case, it is possible to retreat the distal end portion 134 of the insertion member 130 to the lumen 118 of the puncturing needle 110 to make the hook 164 and the distal end portion side surface of the extendedportion 172 abut again and return to initial positions by gripping the vicinity of the end surface 137 and moving the insertion member 130 against the urging force in a state in which the position of the operating unit 140 at the proximal end portion is fixed, for example.
As described above, the first embodiment includes the pullout preventing device which prevents the needle tip (puncturing needle) from being pulled out from the puncturing site after the load of the puncturing disappears and puncturing is completed. Thus, it is not necessary to prevent the needle tip from being pulled out by way of deep puncturing, for example. Further, the needle tip is prevented from being pulled out in conjunction with protruding movement of the distal end portion of the insertionmember from the lumen of the puncturing needle, so that operability is good. Consequently, it is possible to provide a Veress needle which can easily prevent the needle tip from being pulled out from the puncturing site.

Next, the second embodiment will be described. In addition, members having the same functions as those in the first embodiment will be assigned similar reference numerals, and descriptions of the members will be suitably omitted to prevent duplication.

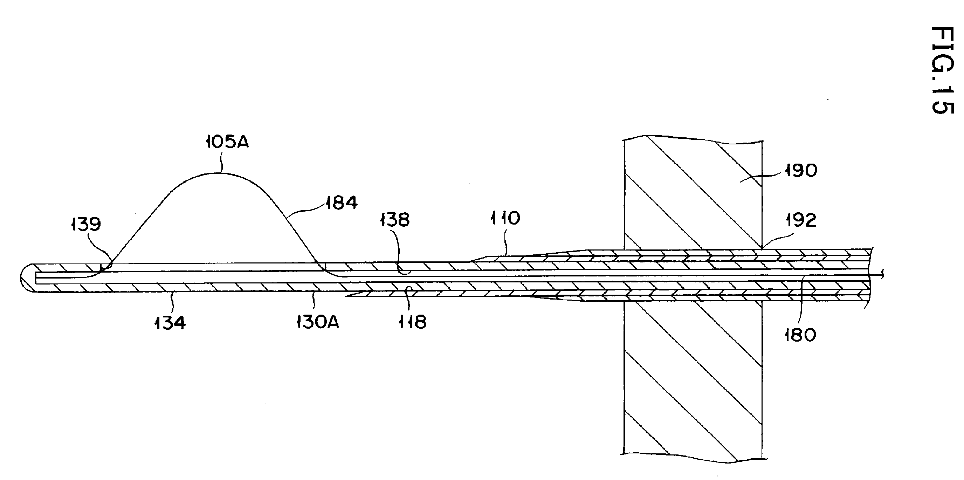

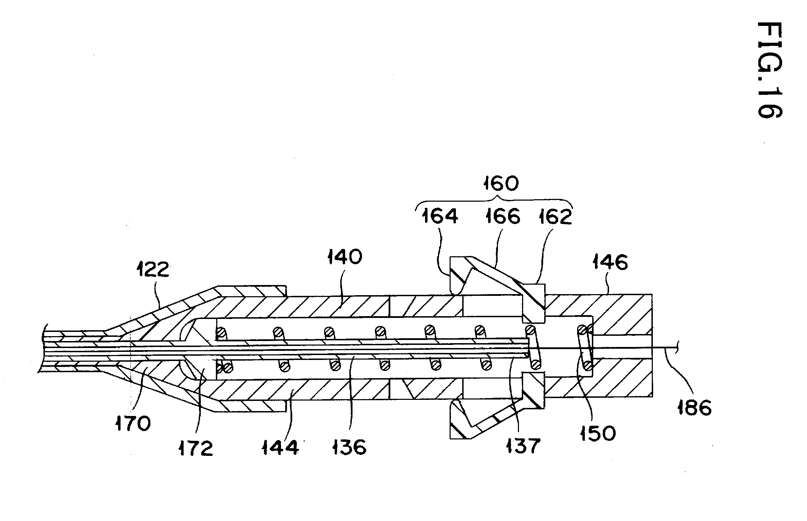

Fig. 15 is a cross-sectional view of assistance in explaining a distal end portion of a Veress needle according to the second embodiment, and Fig. 16 is a cross-sectional view of assistance in explaining an operating unit at the proximal end portion of the Veress needle according to the second embodiment.

The second embodiment relates to a configuration of the pullout preventing device, and roughly differs from the first embodiment in applying the extension of a wire.

Specifically, an insertion member 130A is in a hollow shape, and has a lumen 138 in which a wire (second insertion member) 180 is disposed. A distal end portion 134 of the insertion member 130A is closed, and a lateral opening 139 which communicates with the lumen 138 is formed. The wire 180 has a distal end portion 184 which is connected to a closed portion of the distal end portion 134, and a proximal end portion 186 which passes in the lumen 138 and extends to an outside of the operating unit 140 at the proximal end portion.

The distal end portion 184 of the wire 180 is in a bent shape and is inserted in the lumen 138 of the insertion member 130A in an elastically deformed state and is adapted to be restored to the bent shape before elastic deformation and is caught on the inner surface of a puncturing site by expanding (protruding) from the lateral opening 139 of the insertion member 130A which protrudes from a lumen 118 of a puncturing needle 110 after a load of the puncturing disappears.

In the second embodiment as described above, in the second embodiment, the pullout preventing device is formed by the distal end portion of the wire, and the pullout preventing device can adopt a simple structure as with the first embodiment.

Next, the third embodiment will be described.

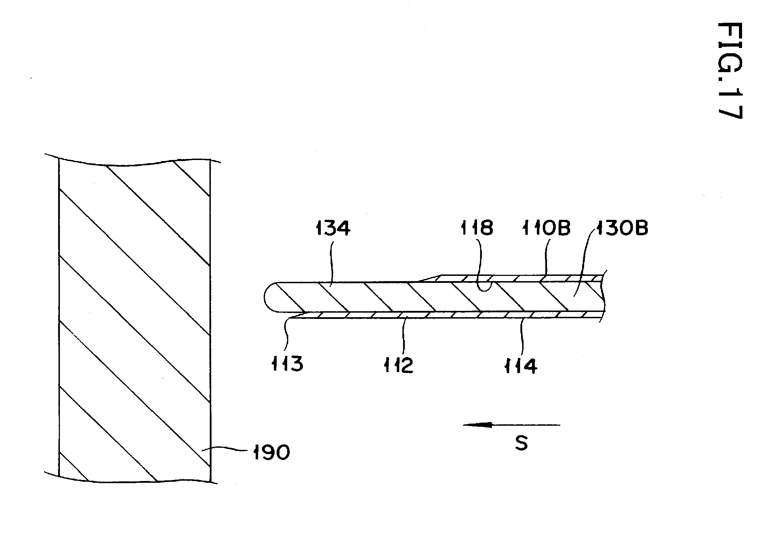

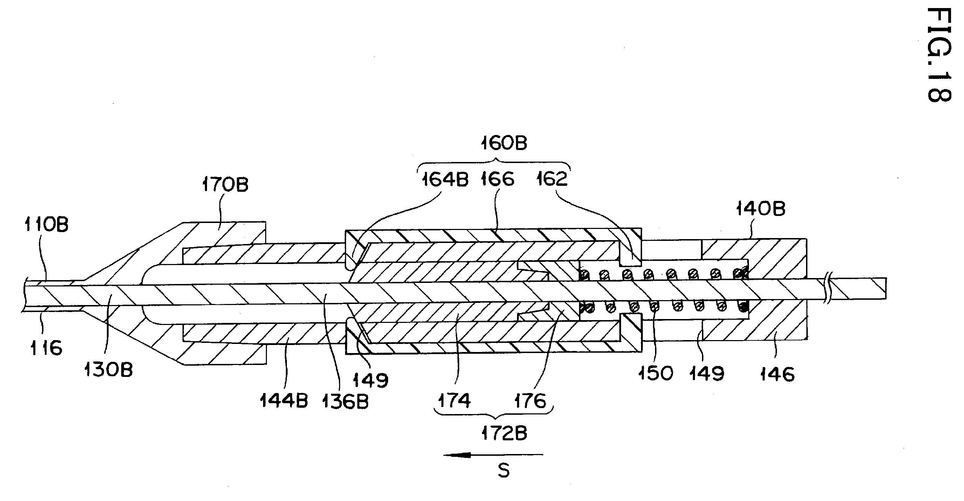

Fig. 17 is a cross-sectional view of assistance in explaining a distal end portion of a Veress needle according to the third embodiment, and Fig. 18 is a cross-sectional view of assistance in explaining an operating unit at the proximal end portion of the Veress needle according to the third embodiment.

The third embodiment roughly differs from the first embodiment in that a tubular sleeve is not provided, an insertion member is formed by a guide wire and the guide wire can be securely indwelled at a puncturing site.

Specifically, the Veress needle according to the third embodiment has a puncturing needle 110B, an insertion member 130B, a pullout preventing device 105B and an operating unit 140 at the proximal end portion.

The puncturing needle 110B has a proximal end portion 116 to which a needle hub 170B is connected. The needle hub 170B is separably attached to the operating unit 140B at the proximal end portion.

The insertion member 130B has a proximal end portion 136B which is extended inside the operating unit 140 at the proximal end portion. To the proximal end portion 136B, a fixing connector 172B which forms an extended portion which protrudes in a direction which crosses an axial direction S of the insertion member 130B is detachably fixed.

The fixing connector 172B is an extended portion of the proximal end portion of the insertion member which is formed by detachable separate member, and has a connector main body 174 and a connector cap 176. The connector main body 174 and the connector cap 176 are slidably disposed inside the operating unit 140 at the proximal endportion, and the proximal end portion 136B of the insertion member 130B is inserted (penetrated) thereinto. Inaddition, the connector main body 174 is adapted to be able to give a fastening force to the insertion member 130B when the connector main body 174 is slidably attached to the proximal end portion 136B and the connector cap 176 rotates and engages with to the connector main body 174, for example.

Thereby, it is, for example, possible to allow the insertion member 130B to slide by means of decreasing the fastening force (or by eliminating the fastening force) when the insertion member 130B is inserted in the fixing connector 172B or the insertion member 130B is detached from the fixing connector 172B, and to stop the insertion member 130B from sliding (to fix the fixing connector 172B to the insertion member 130B) by means of increasing the fastening force and increasing a resistance upon movement of the insertion member 130B upon use.

In addition, the insertion member 130B which is formed by the guide wire adopts a multilayer structure, and has a single wire (base material) in a straight shape and a resin layer which covers a surface of the wire. The wire is made of nickel titanium, and the resin layer has a lower layer which is closely attached to the wire and a fluorine resin layer which forms an outer shell. The insertion member 130B is not limited to the above configuration in particular and can also be formed by a coil member made of nickel titanium or stainless steel, and a urethane resin layer is also applicable as the outer shell of the resin layer.

The operating unit 144B at the proximal end portion has a distal end portion to which the needle hub 170B is detachable, and has an urging device 150B and a locking device 160B disposed therein.

The urging device 150B is formed by an elastic member (spring) which urges the fixing connector 172B toward a distal end side, and is disposed between the connector cap 176 and the cap portion 146 of the operating unit 140B at the proximal end portion. The hook 164B of the locking device 160B is disposed closer to the distal end portion side than the connector main body 174, and abuts on a distal end portion side surface of the connector main body 174 and locks movement of the insertion member 130 to which the fixing connector 172B is fixed in an urging direction (in the axial direction S leading to the distal end portion) for use of defining an initial position. In addition, the fixing connector 172B is adapted to stop when the connector main body 174 abuts on an inner surface of the needle hub 170B. Hence, the amount of protrusion of the insertion member 130B is defined by a distance between the distal end side surface of the connector main body 174 and the inner surface of the needle hub 170B at the initial position.

Next, a method of using the Veress needle according to the third embodiment will be described.

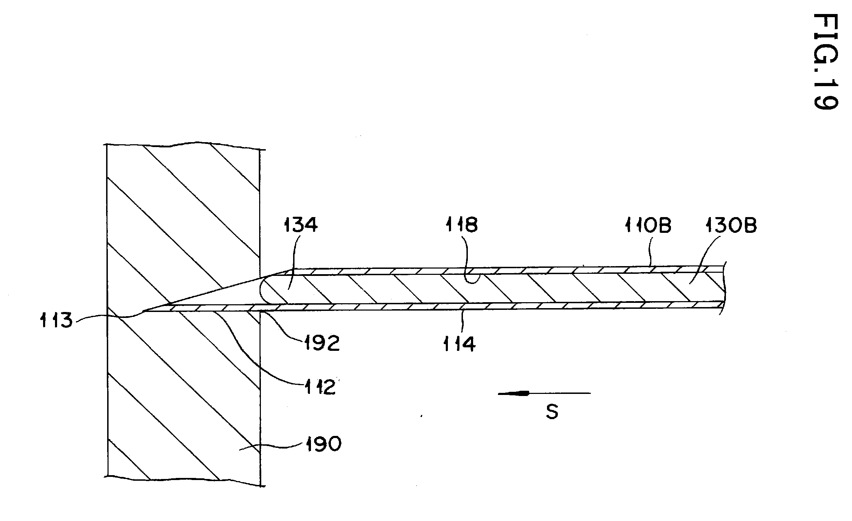

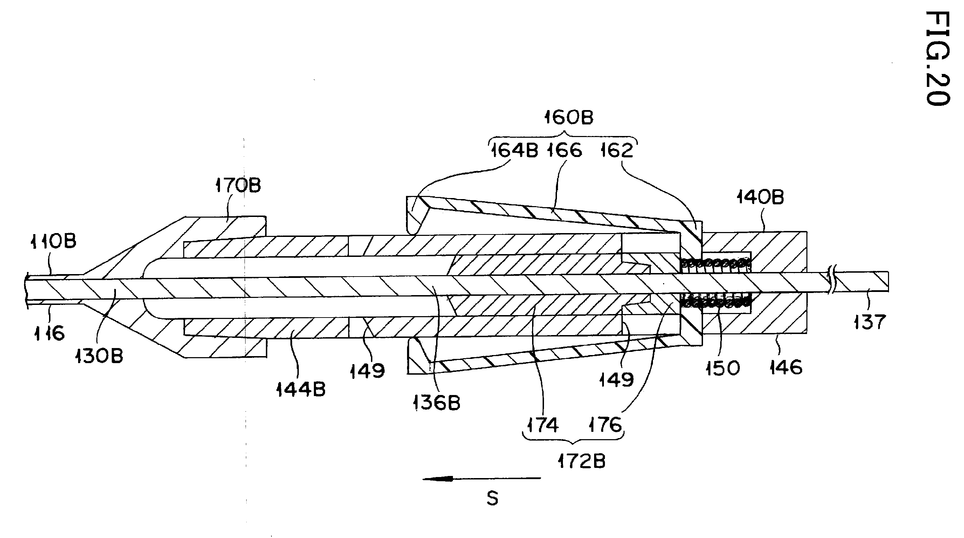

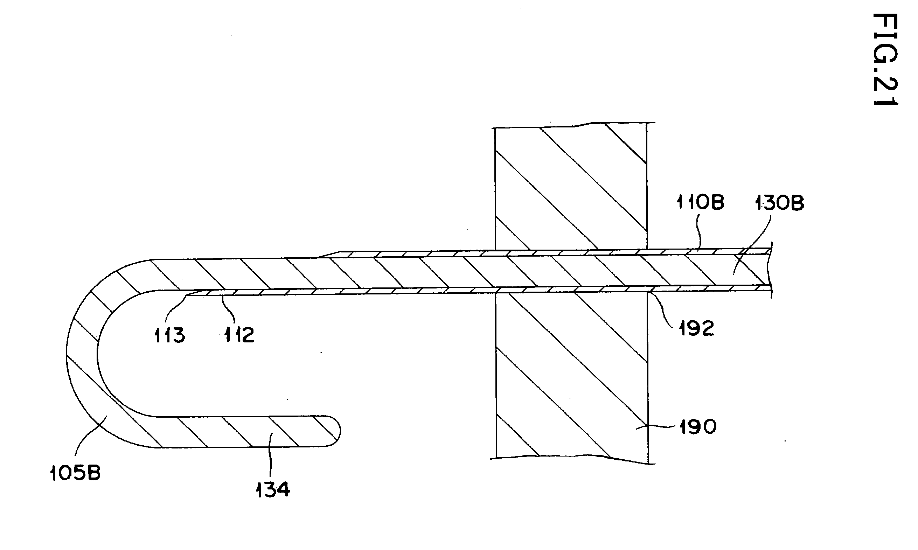

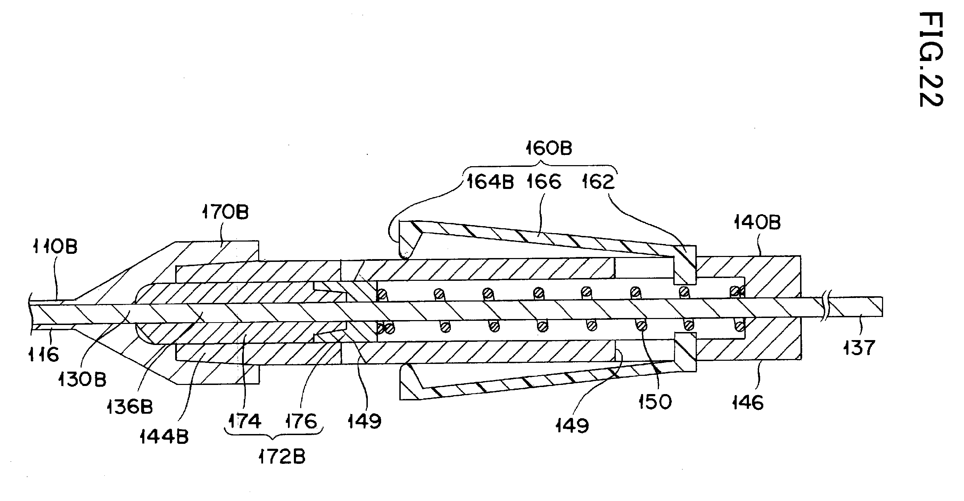

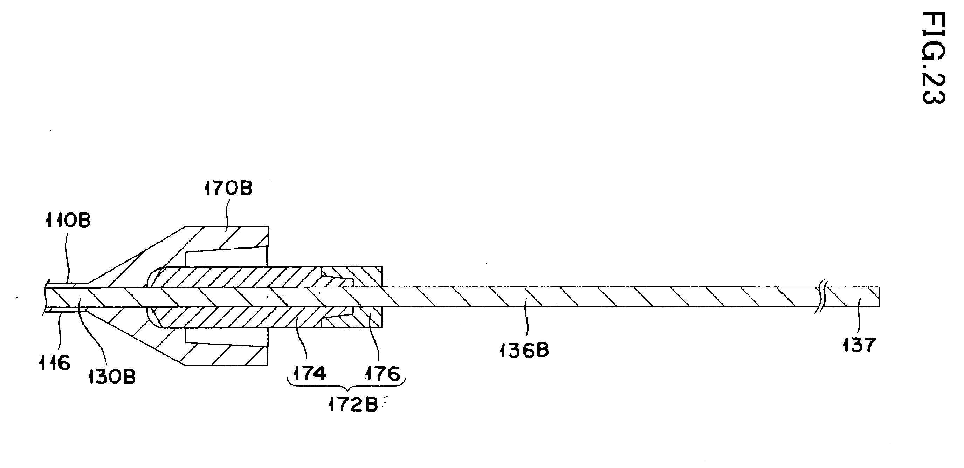

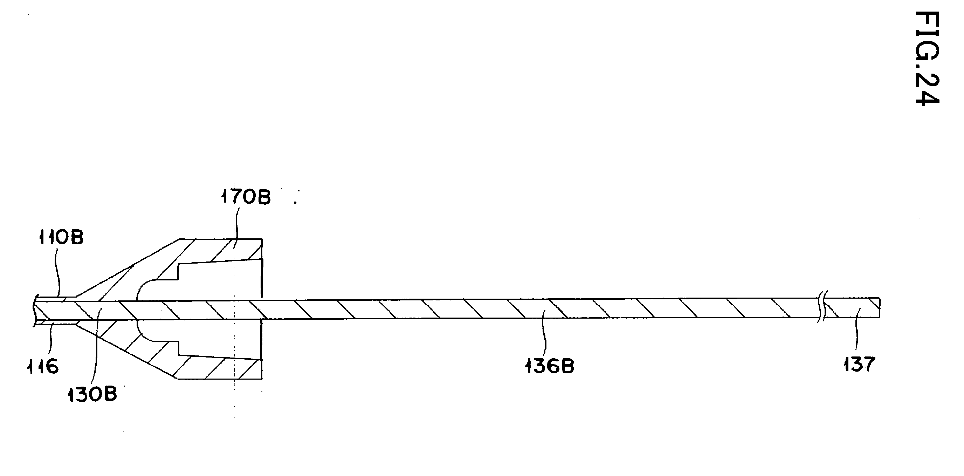

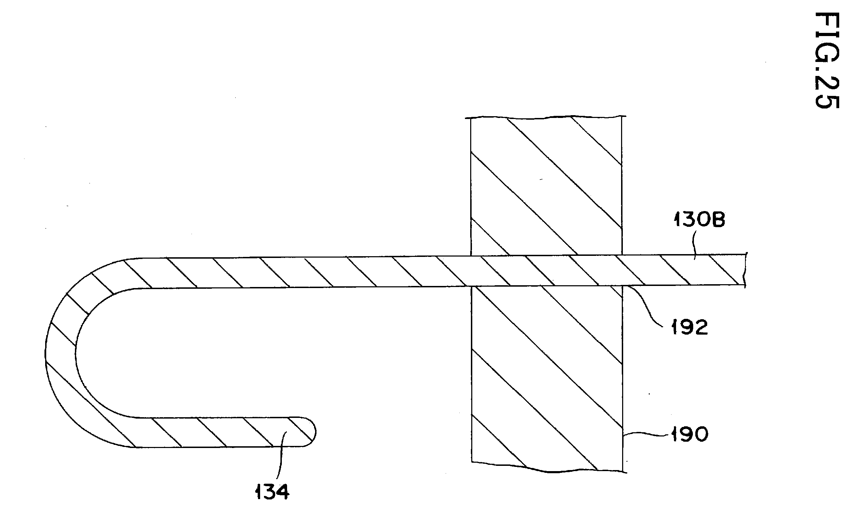

Figs. 19 and 20 are cross-sectional views of assistance in explaining the distal end portion and the operating unit at the proximal end portion upon start of puncturing, Figs. 21 and 22 are cross-sectional views of assistance in explaining the distal end portion and the operating unit at the proximal end portion upon completion of puncturing, Fig. 23 is a cross-sectional view of assistance in explaining separation of the operating unit at the proximal end portion, Fig. 24 is a cross-sectional view of assistance in explaining separation of the fixing connector and Fig. 25 is a cross-sectional view of assistance in explaining how the guide wire is indwelled.

When the Veress needle is applied to the transvaginal hydrolaparoscopy, preliminary arrangements and insertion of the distal end portion of the Veress needle into the vagina are conducted as with the first embodiment.

Further, when abutting on a wall portion 190, the distal end portion 134 of the insertion member 130B retreats to the lumen 118 of the puncturing needle 110B against an urging force of the urging device 150 based on a resistance resulting from the aforementioned abutting. Thereby, the distal end portion 114 of the puncturing needle 110B is exposed and the wall portion 190 of the vaginal wall is punctured by a needle tip 112 of the distal end portion 114 as illustrated in Fig. 19.

At this point, the proximal end portion side surface of the connector cap 176 of the fixing connector 172B abuts on a sliding portion 162 of the locking device 160B, and moves (drives) the sliding portion 162 along the proximal end portion 136B of the insertion member 130B. Thereby, hooks 164B of the locking device 160B move accompanying the sliding portion 162, are pulled out from an opening 149 (separated from the insertion member 130B), are elastically deformed, and abut on (get on) the outer periphery of the operating unit 140B at the proximal end portion. Thus, the lock of the fixing connector 172B (connector main body 174) is unlocked as illustrated in Fig. 20.

Then, the needle tip 112 penetrates the wall portion 190 and the resistance resulting from the abutting on the wall portion 190 disappears. Thus, the distal end portion 134 of the insertion member 130B protrudes from the distal end portion 114 of the puncturing needle 110B based on the urging force of the urging device 150 and guards the needle tip 112. Consequently, it is possible to prevent a damage on an organ positioned on an inner side of a puncturing site 192 of the wall portion 190.

At this point, the lock of the fixing connector 172B is unlocked (the hook 164B does not abut on the distal end portion side surface of the connector main body 174). Therefore, the force of the urging device 150 contracted on the proximal end side is released and the fixing connector 172B moves and abuts on the inner surface of the needle hub 170B as illustrate in Fig. 22.

Thereby, the distal end portion 134 of the insertion member 130B protrudes beyond the initial position, and is restored to a bent shape before elastic deformation and is caught on the inner surface of the puncturing site 192, so that the needle tip 112 is prevented from being pulled out from the puncturing site 192 as illustrated in Fig. 21.

Next, the needle hub 170B and the operating unit 140B at the proximal end portion are disconnected, and the operating unit 140B at the proximal end portion is retreated in a state in which the position of the needle hub 170B is fixed. Thereby, the operating unit 140B at the proximal endportion is separated as illustrated in Fig. 23.

After that, the fastening force with respect to the insertionmember 130B is weakened (or eliminated) with respect to the insertion member 130B by rotating the connector cap 176, and the fixing connector 172B is slid (moved) along the proximal end portion 136B of the insertion member 130B and is detached from an end surface 137 of the proximal end portion 136B. Thereby, the fixing connector 172B is separated as illustrated in Fig. 24.

Then, the needle hub 170B is retreated in a state in which the position of the insertion member 130B is fixed. Thereby, only the insertion member 130B is indwelled in the puncturing site 192 of the wall portion 190 as illustrated in Fig. 25.

The insertionmember 130B which is formed by the indwelled guide wire is used for, for example, injection of physiological salt solution, insertion of an endoscope and insertion of a catheter which has a lumen used for medical treatment, etc. After use, bleeding from the puncturing site 192 is stopped as after-treatment.

In the third embodiment as described above, the insertion member is formed by the guide wire, so that the guide wire can be securely indwelled in a puncturing site. In addition, the tubular sleeve according to the first embodiment and the second embodiment can be suitably incorporated in the third embodiment.

The present invention is not limited to the above embodiments, and can be variously modified within the scope of the claims. For example, the present invention is also applicable as a pneumoperitoneum needle for introducing carbon dioxide into an abdominal cavity. The pullout preventing device is not limited to the embodiment in which the pullout preventing device is formed by the distal end portion of the insertion member and the embodiment in which the pullout preventing device is disposed at the distal end portion. It is also possible to form a checking device for visually checking completion of the puncturing as an opening which is disposed on the outer periphery of the operating unit at the proximal end portion, and visually check movement of an extended portion (connector) through the opening. It is also possible to make a puncturing needle in a hollow shape to allow a catheter, an endoscope, and the like to be inserted therein.

This application is based on Japanese Patent Application No. 2011-033414 filed on February 18, 2011, the contents of which are hereby incorporated by reference.

### Explanation of reference signs

- 100: VERESS NEEDLE,
- 105, 105AB, 105B: PULLOUT PREVENTING DEVICE,
- 110, 110B: PUNCTURING NEEDLE,
- 112: NEEDLE TIP,
- 113: BEVEL TIP SIDE,
- 114: DISTAL END PORTION,
- 116: PROXIMAL END PORTION,
- 118: LUMEN,
- 120: TUBULAR SLEEVE
- 122: SLEEVE HUB,
- 128: LUMEN,
- 130, 130A, 130B: INSERTION MEMBER,
- 134: DISTAL END PORTION,
- 136, 136B: PROXIMAL END PORTION,
- 137: END SURFACE,
- 138: LUMEN,
- 139: LATERAL OPENING,
- 140, 140B: OPERATING UNIT AT PROXIMAL END PORTION,
- 144, 144B: DISTAL END PORTION,
- 146: CAP PORTION,
- 149: OPENING,
- 150, 150B: URGING DEVICE,
- 160, 160B: LOCKING DEVICE,
- 162: SLIDING PORTION,
- 164, 164B: HOOK,
- 166: ARM,
- 170, 170B: NEEDLE HUB,
- 172: EXTENDED PORTION,
- 172B: FIXING CONNECTOR,
- 174: CONNECTOR MAIN BODY,
- 176: CONNECTOR CAP,
- 180: WIRE,
- 184: DISTAL END PORTION,
- 186: PROXIMAL END PORTION,
- 190: WALL PORTION,
- 192: PUNCTURING SITE,
- S: AXIAL DIRECTION.

## Claims

1. A Veress needle comprising:
a hollow puncturing needle which comprises a distal end portion with a needle tip and a lumen;
an insertion member which is slidably inserted in said lumen;
an urging device which urges said insertion member such that, before puncturing is performed by means of said needle tip and after the puncturing is completed, a distal end portion of said insertion member protrudes from said needle tip and, the distal end portion of said insertion member is retreated to said lumen based on a load of the puncturing upon the puncturing by means of said needle tip; and
a pullout preventing device which, after the load of the puncturing disappears, prevents said needle tip frombeing pulled out from a puncturing site in conjunction with protruding movement of the distal end portion of said insertion member from said lumen.

2. The Veress needle according to claim 1, further comprising an operating unit which is positioned on a proximal end portion of said puncturing needle and at which said urging device is disposed, said operating unit at the proximal end portion comprising a locking device which locks movement of said insertion member, wherein
said locking device is adapted to lock the movement of said insertion member in an urging direction to define an initial position of the distal end portion of said insertion member before the puncturing by means of said needle tip, and, when the distal end portion of said insertion member retreats to said lumen, unlocks the lock and releases an urging force of said urging device, and
said pullout preventing device prevents said needle tip from being pulled out from the puncturing site based on the protruding movement of the distal end portion of said insertion member beyond the initial position due to the released urging force after the load of the puncturing disappears.

3. The Veress needle according to claim 2, wherein said locking device comprises a hook which locks the movement of said insertion member in the urging direction by means of engaging with said insertion member, and unlocks the lock by means of moving away from said insertion member based on retreatingmovement of the distal end portion of said insertion member to said lumen.

4. The Veress needle according to claim 3, wherein a proximal end portion of said insertion member comprises an extended portion which is disposed closer to a proximal end portion side than said hook and which protrudes in a direction which crosses an axial direction of said insertion member, wherein
said locking device further comprises:
a sliding portion which is disposed closer to the proximal end portion side than said extended portion, and which is driven by abutting a proximal end portion side surface of said extended portion which moves based on the retreating movement of the distal end portion of said insertion member to said lumen, and moves along the proximal end portion of said insertion member; and
an arm which is extended from an outer periphery of said operating unit at the proximal end portion, and which connects said sliding portion and said hook through an opening formed in said operating unit at the proximal end portion, wherein
said hook engages with said insertion member by means of abutting on a distal end portion side surface of said extended portion, and moves accompanying the movement of said sliding portion, and then is separated from said insertion member and disengages the engagement.

5. The Veress needle according to claim 4, wherein said pullout preventing device is formed by the distal end portion of said insertion member, and
the distal end portion of said insertion member is in a bent shape, and is inserted in said lumen in an elastically deformed state, protrudes from said lumen after the puncturing is completed, then is restored to the bent shape before elastic deformation and is caught on an inner surface of the puncturing site.

6. The Veress needle according to claim 4, wherein said insertion member is hollow and comprises a lumen in which a second insertion member is inserted,
the distal end portion of said insertion member comprises a lateral opening which communicates with said lumen, and
said pullout preventing device is formed by a distal end portion of said second insertion member, wherein
the distal end portion of said second insertion member is in a bent shape, and is inserted in said lumen of said insertion member in an elastically deformed state, is extended from the lateral opening of said insertion member which protrudes from said lumen of said puncturing needle after the load of the puncturing disappears, then is restored to the bent shape before elastic deformation and is caught on the inner surface of the puncturing site.

7. The Veress needle according to claims 5 or 6, wherein said insertion member is detachably attached to said operating unit at the proximal end portion.

8. The Veress needle according to one of claims 4-7, further comprising a hollow tubular sleeve in which said puncturing needle is slidably inserted, wherein said tubular sleeve is detachably attached to said operating unit at the proximal end portion.

9. The Veress needle according to one of claims 4-8, wherein said operating unit at the proximal end portion comprises a checking device for checking completion of the puncturing visually.

10. The Veress needle according to claim 9, wherein said checking device is formed by an end surface of the proximal end portion of said insertion member, said end surface adapted to protrude outward from a proximal end portion of said operating unit at the proximal end portion when being at the initial position, and be accommodated in said operating unit at the proximal end portion when the puncturing is completed.

11. The Veress needle according to claim 4, wherein said insertion member is formed by a guide wire which is adapted to be separably from said puncturing needle and said operating unit at the proximal end portion.

12. The Veress needle according to claim 10, wherein said extended portion at the proximal end portion of said insertion member is formed by a detachable different member.
